# FASCICULE DE BREVET EUROPEEN

(11) **EP 0 667 143 B1**
(45) Date de publication et mention de la délivrance du brevet: **09.10.1996**
(21) Numéro de dépôt: 94402695.4
(22) Date de dépôt: 24.11.1994
(51) Int. Cl.: A61K 7/13

(54) **Composition de teinture d'oxydation des fibres kératiniques comprenant une paraphénylènediamine, une métaphénylènediamine et un paraaminophénol ou un métaaminophénol**
Oxydationsfärbemittel für Keratinfasern, enthaltend ein Paraphenylendiamin, ein Metaphenylendiamin und ein Paraaminophenol oder ein Metaaminophenol
Keratinous fiber oxidation dyeing composition containing a paraphenylenediamine, a metaphenylenediamine and a paraaminophenol or a metaaminophenol

(30) Priorité: 24.01.1994 FR 9400701
(43) Date de publication de la demande: 16.08.1995
(73) Titulaire: L'OREAL, F-75008 Paris (FR)
(72) Inventeur: Audousset, Marie-Pascale, F-92600 Asnieres (FR); Cotteret, Jean, F-78480 Verneuil s/Seine (FR)
(74) Mandataire: Andral, Christophe André Louis

(56) Documents cités:
- EP-A- 0 359 618
- WO-A-93/10744
- DE-A- 3 914 394
- GB-A- 2 018 808
- GB-A- 2 025 958
- GB-A- 2 054 663
- GB-A- 2 078 747

## Description

La présente invention concerne une composition de teinture d'oxydation des fibres kératiniques et en particulier des fibres kératiniques humaines, comprenant en association, au moins une paraphénylènediamine à fonction amine primaire, secondaire ou tertiaire, au moins une métaphénylènediamine, et en outre soit un para-aminophénol soit un méta-aminophénol, de formules décrites dans la suite de la description. Elle concerne également l'utilisation d'une telle composition.

Il est connu de teindre les fibres kératiniques et en particulier les cheveux humains, avec des compositions de teinture contenant des précurseurs de colorants d'oxydation, en particulier des ortho- ou paraphénylènediamines, des ortho-ou para-aminophénols, généralement appelés "bases d'oxydation", et des coupleurs encore appelés modificateurs de coloration, plus particulièrement des métaphénylènediamines, des méta-aminophénols et des métadiphénols, qui permettent de modifier et d'enrichir en reflets les colorations "de fond" obtenues par les produits de condensation des bases d'oxydation.

On recherche activement dans le domaine de la teinture capillaire d'oxydation, des précurseurs de colorants d'oxydation ainsi que des coupleurs capables d'engendrer, lorsqu'ils sont associés, une coloration allant du bleu au bleu-violet ou rouge-violet, ayant une résistance satisfaisante à la lumière, aux lavages, aux intempéries, à la transpiration et aux différents traitements que peuvent subir les cheveux.

Jusqu'ici ces colorations "de fond" ont été obtenues surtout avec des teintures à base de paraphénylènediamine. Mais actuellement, l'utilisation de la paraphénylènediamine est contestée pour des raisons toxicologiques.

Or, la demanderesse vient maintenant de découvrir qu'il est possible d'obtenir de nouvelles teintures non toxiques et résistantes, qui engendrent des colorations intenses allant du bleu au bleu-violet ou rouge-violet, en associant une paraphénylènediamine à fonction amine primaire, secondaire ou tertiaire à une métaphénylènediamine, avec en outre soit un para-aminophénol soit un méta-aminophénol de structures définies ci-après.

Cette découverte est à la base de la présente invention.

La présente invention a donc pour objet une composition de teinture d'oxydation pour fibres kératiniques, en particulier pour fibres kératiniques humaines telles que les cheveux, caractérisée en ce qu'elle comprend, dans un milieu approprié pour la teinture :
(a) au moins un premier précurseur de colorant d'oxydation choisi parmi les paraphénylènediamines de formule (I) suivante : 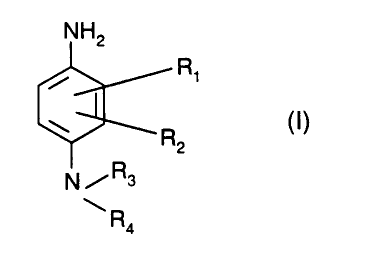 dans laquelle les radicaux R₁, R₂, R₃ et R₄ ont les significations soumises aux conditions suivantes :
   - ou bien R₁ et R₂ représentent un atome d'hydrogène, lorsque R₃ et R₄, identiques, représentent un groupement -(CH₂)ₙ-OH, dans lequel n est égal à 2, 3 ou 4,
   - ou bien R₁, R₂ et R₃ représentent un atome d'hydrogène, lorsque R₄ représente soit un groupement -(CH₂)ₙ-OR₅ dans lequel R₅ désigne un radical méthyle ou éthyle, avec n égal à 2 ou 3, soit un groupement mono- ou dihydroxypropyle,
   - ou bien R₃ et R₄ représentent un atome d'hydrogène, lorsque R₁ et R₂, identiques, représentent un radical méthyle ou éthyle et sont situés en positions 2,3 ; 2,5 ou 2,6 sur le noyau benzénique,
   - ou bien R₂, R₃ et R₄ représentent un atome d'hydrogène, lorsque R₁ représente un radical isopropyle,
   et/ou au moins un des sels d'addition de ces composés de formule (I) avec un acide;
(b) au moins un premier coupleur de type métaphénylènediamine, de formule (II) suivante : 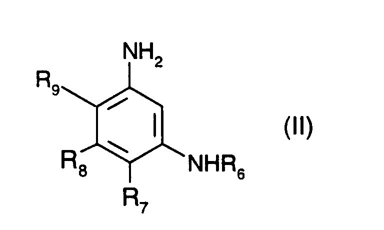 dans laquelle R₆ représente un atome d'hydrogène, un radical alkyle, mono-ou polyhydroxyalkyle, R₇ représente un atome d'hydrogène, un radical alkyle ou monohydroxyalcoxy, R₈ représente un atome d'hydrogène ou un radical alkyle, R₉ représente un radical alcoxy, aminoalcoxy, mono- ou poly-hydroxyalcoxy ou un radical 2,4-diaminophénoxyalcoxy, lesdits radicaux alkyle ou alcoxy ci-dessus contenant de 1 à 4 atomes de carbone, lesdits radicaux mono- ou polyhydroxyalkyle et mono- ou poly-hydroxyalcoxy ci-dessus désignant des radicaux alkyle ou alcoxy contenant de 2 à 3 atomes de carbone et comportant de 1 à 3 groupements hydroxyle, étant entendu qu'au moins un des radicaux R₇ ou R₈ désigne un atome d'hydrogène,
   et/ou au moins l'un des sels d'addition de ces composés de formule (II) avec un acide;
(c)
   - soit au moins un deuxième précurseur de colorant d'oxydation, de type paraaminophénol, de formule (III) suivante : 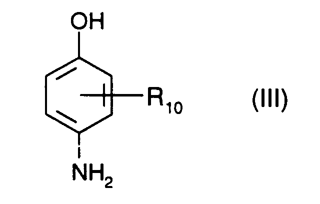 dans laquelle R₁₀ représente un radical alkyle, monohydroxyalkyle ou monoalcoxyalkyle, les radicaux alkyle et alcoxy ci-dessus contenant de 1 à 4 atomes de carbone,
      et/ou au moins l'un des sels d'addition de ces composés de formule (III) avec un acide;
   - soit au moins un deuxième coupleur, de type méta-aminophénol, de formule (IV) suivante : 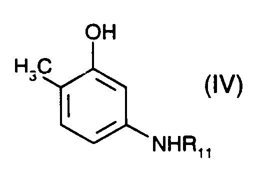 dans laquelle R₁₁ représente un atome d'hydrogène, un radical alkyle contenant 1 ou 2 atomes de carbone ou un radical β-hydroxyalkyle contenant de 2 à 3 atomes de carbone,
      et/ou au moins l'un des sels d'addition de ces composés de formule (IV) avec un acide ;
Sont toutefois exclues du cadre de la présente invention :
(i) les compositions renfermant simultanément des composés de formules (I), (II) et (IV) pour lesquelles R₁, R₂ désignent le radical méthyle en position 2 sur le noyau benzénique, R₃, R₄, R₆, R₇, R₈ désignent un atome d'hydrogène, R₉ désigne un radical aminoéthyloxy, et R₁₁ un groupement β-hydroxyéthyle,
ainsi que (ii) les compositions renfermant simultanément des composés de formules (I), (II) et (IV) pour lesquelles R₁, R₂, R₃, R₆, R₇, R₈, R₁₁ désignent l'hydrogène, R₄ désigne un radical méthoxyéthyle, et R₉ désigne un groupement β-hydroxyéthyloxy.

Les nouvelles teintures ainsi obtenues permettent d'aboutir à des colorations intenses, allant du bleu au bleu-violet ou rouge-violet, non toxiques et résistant particulièrement bien à la lumière, aux lavages, aux intempéries, à la transpiration et aux différents traitements que peuvent subir les cheveux. Elles sont tout particulièrement très résistantes aux shampooings.

Un autre objet de la présente invention porte sur la composition prête à l'emploi, contenant les différents agents utilisés pour la teinture des fibres kératiniques définis ci-dessus et un agent oxydant.

L'invention vise également un procédé de teinture des fibres kératiniques, et en particulier des fibres kératiniques humaines telles que les cheveux, consistant à appliquer sur ces fibres au moins une composition (A) contenant dans un milieu approprié pour la teinture les précurseurs de colorants d'oxydation et les coupleurs tels qu'ils ont été définis ci-avant, la couleur étant révélée à pH alcalin neutre ou acide à l'aide d'un agent oxydant qui est ajouté juste au moment de l'emploi à la composition (A) ou qui est présent dans une composition (B) appliquée simultanément ou séquentiellement de façon séparée.

L'invention a également pour objet des dispositifs de teinture ou "kits" à plusieurs compartiments dont le premier compartiment contient au moins les précurseurs de colorants d'oxydation et coupleurs définis ci-avant, et le deuxième, un agent oxydant.

D'autres caractéristiques, aspects, objets et avantages de l'invention apparaîtront encore plus clairement à la lecture de a description et des exemples qui suivent.

Les sels d'acide utilisés selon l'invention sont choisis de préférence parmi les chlorhydrates, les sulfates, les bromhydrates et les tartrates.

Selon l'invention, à titre de premier précurseur de colorant d'oxydation, on préfère utiliser une paraphénylènediamine de formule (I) qui est choisie parmi les composés suivants :
la 2,6-diméthylparaphénylènediamine ;
la 2,6-diéthylparaphénylènediamine ;
la 2,3-diméthylparaphénylènediamine ;
la 2,5-diméthylparaphénylènediamine ;
la 2-isopropylparaphénylènediamine ;
la N-(β-méthoxyéthyl) paraphénylènediamine ;
la N,N-di-(β-hydroxyéthyl) paraphénylènediamine et leurs sels.

Dans la formule (II) définissant le coupleur de type métaphénylènediamine, on préfère les composés pour lesquels R₆ désigne un atome d'hydrogène, un radical alkyle ou hydroxyalkyle, R₇ et R₈ désignent un atome d'hydrogène, R₉ désigne un radical alcoxy, aminoalcoxy, hydroxyalcoxy ou poly-hydroxyalcoxy, les radicaux alkyle et alcoxy de tous ces composés contenant de 1 à 4 atomes de carbone, a l'exception du groupement poly-hydroxyalcoxy pour lequel le radical alcoxy comporte de 2 à 4 atomes de carbone et de 1 à 3 radicaux hydroxyle.

Parmi de tels composés, on peut citer : le 1-(β-hydroxyéthyloxy) 2,4-diamino benzène, le 1-méthoxy 2-amino 4-(β-hydroxyéthylamino) benzène, le 1-(β-aminoéthyloxy) 2,4-diamino benzène, le 1-(β-hydroxyéthyloxy) 2-amino 4-(méthylamino) benzène et leurs sels.

D'autres composés de formule (II) intéressants peuvent être choisis parmi le 1-éthyl 2-méthoxy 3,5-diamino benzène, le 1-méthyl 2-méthoxy 3,5-diamino benzène, le 1,3-bis-(2,4-diaminophénoxy) propane, le 1,3-bis-(2,4-diaminophénoxy) méthane, le 1,2-bis-(2,4-diaminophénoxy) éthane, le 1,3-diamino 4,6-bis-(β-hydroxyéthyloxy) benzène, le 1-éthoxy 2,4-diamino 5-méthyl benzène, le 1-méthyl 2,4-diamino 5-(β-hydroxyéthyloxy) benzène et leurs sels.

Plus particulièrement encore, selon la présente invention, on préfère mettre en oeuvre les métaphénylènediamines de formule (II) correspondant aux composés suivants :
le 1-(β-hydroxyéthyloxy) 2,4-diamino benzène ;
le 1-méthoxy 2-amino 4-(β-hydroxyéthylamino) benzène et leurs sels.

Dans la formule (III) définissant le précurseur de colorant d'oxydation de type paraaminophénol, le radical alkyle désigne de préférence les radicaux méthyle, éthyle, propyle, isopropyle, butyle, sec-butyle, tertiobutyle, et les radicaux monohydroxyalkyle et monoalcoxyalkyle désignent de préférence les radicaux suivants : -CH₂OH ; -CH₂-CH₂OH ; -CH₂-CHOH-CH₂OH ; -CH₂-CHOH-CH₃ ou encore -CH₂ O CH₃.

Plus particulièrement encore, selon la présente invention, on préfère mettre en oeuvre les paraaminophénols de formule (III) correspondant aux composés suivants :
le 3-méthyl 4-aminophénol,
le 3-éthyl 4-aminophénol,
le 3-hydroxyméthyl 4-aminophénol,
le 2-méthyl 4-aminophénol,
le 2-hydroxyméthyl 4-aminophénol,
le 3-méthoxyméthyl 4-aminophénol,
le 2-méthoxyméthyl 4-aminophénol et leurs sels.

Dans la formule (IV) définissant le coupleur de type méta-aminophénol, le radical alkyle désigne de préférence les radicaux méthyle ou éthyle, et le radical β-hydroxyalkyle désigne de préférence -CH₂-CH₂OH ou -CH₂-CH₂-CH₂OH.

Plus particulièrement encore, selon la présente invention, on préfère mettre en oeuvre les méta-aminophénols de formule (IV) correspondant aux composés suivants :
le 2-méthyl 5-aminophénol,
le 2-méthyl 5-N-méthylaminophénol,
le 2 méthyl 5-N-éthylaminophénol,
le 2-méthyl 5-N-(β-hydroxyéthylamino) phénol,
le 2-méthyl 5-N-(β-hydroxypropylamino) phénol et leurs sels.

Des compositions de teinture d'oxydation particulièrement préférées dans le cadre de la présente invention sont celles qui renferment, dans un milieu approprié pour la teinture, au moins :
- un composé (a) à titre de premier précurseur d'oxydation,
- un composé (b) à titre de premier coupleur,
- un composé (c) à titre soit de second précurseur de colorant d'oxydation, soit de second coupleur,
chacun de formules respectives (I), (II), (III), et (IV) telles que définies ci-avant, ces composés (a), (b) et (c) étant choisis parmi les composés suivants :
(a)1: la 2,6-diméthylparaphénylènediamine,
(a)2: la 2,6-diéthylparaphénylènediamine,
(a)3: la 2,3-diméthylparaphénylènediamine,
(a)4: la 2-isopropylparaphénylènediamine,
(a)5: la N,N-di-(β-hydroxyéthyl) paraphénylènediamine
(a)6: la N-(β-méthoxyéthyl) paraphénylènediamine,
(b)1: le 1-(β-hydroxyéthyloxy) 2,4-diamino benzène,
(b)2: le 1-méthoxy 2-amino 4-(β-hydroxyéthylamino) benzène,
(c)1: le 2-méthyl 5-N-(β-hydroxyéthylamino) phénol,
(c)2: le 2-méthyl 5-aminophénol,
(c)3: le 3-méthyl 4-aminophénol et leurs sels.

Lesdites compositions préférées selon l'invention correspondent donc aux associations suivantes :
(a)1+(b)1+(c)1 ; (a)1+(b)1+(c)2 ; (a)1+(b)1+(c)3 ; (a)2+(b)1+(c)1 ; (a)2+(b)1+(c)2 ;
(a)2+(b)1+(c)3 ; (a)3+(b)1+(c)1 ; (a)3+(b)1+(c)2 ; (a)3+(b)1+(c)3 ; (a)4+(b)1+(c)1 ;
(a)4+(b)1+(c)2 ; (a)4+(b)1+(c)3 ; (a)5+(b)1+(c)1 ; (a)5+(b)1+(c)2 ; (a)5+(b)1+(c)3 ;
(a)6+(b)1+(c)1 ; (a)6+(b)1+(c)3 ; (a)1+(b)2+(c)1 ; (a)1+(b)2+(c)2 ; (a)1+(b)2+(c)3 ;
(a)2+(b)2+(c)1 ; (a)2+(b)2+(c)2 ; (a)2+(b)2+(c)3 ; (a)3+(b)2+(c)1 ; (a)3+(b)2+(c)2 ;
(a)3+(b)2+(c)3 ; (a)4+(b)2+(c)1 ; (a)4+(b)2+(c)2 ; (a)4+(b)2+(c)3 ; (a)5+(b)2+(c)1 ;
(a)5+(b)2+(c)2 ; (a)5+(b)2+(c)3 ; (a)6+(b)2+(c)1 ; (a)6+(b)2+(c)2 ; (a)6+(b)2+(c)3.

Dans les compositions de teinture d'oxydation selon l'invention :
- la quantité de premier précurseur de colorant d'oxydation de formule (I) ou ses sels peut varier entre 0,01% et 10% en poids et de préférence entre 0,05% et 5% en poids par rapport au poids total de la composition ;
- la quantité de premier coupleur de formule (II) ou ses sels peut varier entre 0,001 et 3 % en poids et de préférence entre 0,005 et 2 % en poids par rapport au poids total de la composition ;
- la quantité de second précurseur de colorant d'oxydation de formule (III) ou ses sels peut varier entre 0,01 % et 5 % en poids et de préférence entre 0,05 % et 3 % en poids par rapport au poids total de la composition;
- et la quantité de second coupleur de formule (IV) ou ses sels peut varier entre 0,005 % et 5 % en poids et de préférence entre 0,01 % et 3 % en poids par rapport au poids total de la composition.

L'agent oxydant est choisi de préférence parmi le peroxyde d'hydrogène, le peroxyde d'urée, les bromates de métaux alcalins, les persels tels que les perborates et les persulfates. L'utilisation du peroxyde d'hydrogène est particulièrement préférée.

La composition (A), qui renferme l'association des colorants telle que décrite ci-dessus, peut avoir un pH compris entre 3 et 11, qui peut être ajusté à la valeur choisie au moyen soit d'agents alcalinisants habituellement utilisés en teinture des fibres kératiniques, tels que l'ammoniaque, les carbonates alcalins, les alcanolamines, par exemple les mono-, di - et triéthanolamines ainsi que leurs dérivés, les hydroxydes de sodium ou de potassium, les composés de formule : 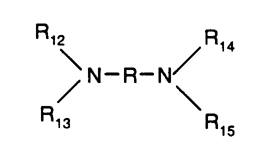
dans laquelle R est un reste propylène éventuellement substitué par un groupement hydroxyle ou un radical alkyle en C₁-C₄ ; R₁₂ R₁₃, R₁₄ et R₁₅ simultanément ou indépendamment l'un de l'autre, représentent un atome d'hydrogène, un radical alkyle en C₁ - C₄ ou hydroxyalkyle en C₁ - C₄,
soit au moyen d'agents acidifiants classiques, tels que les acides minéraux ou organiques comme par exemple les acides chlorhydrique, tartrique, citrique et phosphorique.

Le pH de la composition (B) renfermant l'agent oxydant tel que défini ci-dessus, est tel qu'après un mélange avec la composition (A), le pH de la composition appliquée sur les fibres kératiniques humaines varie de préférence entre 3 et 11. Il est ajusté à la valeur désirée à l'aide d'agents acidifiants ou éventuellement alcalinisants bien connus de l'état de la technique, notamment tels que ceux décrits ci-dessus.

La composition oxydante (B) est de préférence constituée par une solution d'eau oxygénée.

Selon un mode de réalisation préféré du procédé de teinture de l'invention, on mélange, au moment de l'emploi, la composition de teinture (A) décrite ci-dessus avec une solution oxydante en une quantité suffisante pour développer une coloration. Le mélange obtenu est ensuite appliqué sur les fibres kératiniques humaines et on laisse poser pendant 5 à 40 minutes, de préférence 15 à 30 minutes, après quoi on rince, on lave au shampooing, on rince à nouveau et on sèche.

Les compositions de teinture peuvent encore contenir, en plus des colorants définis ci-dessus, d'autres coupleurs et/ou des colorants directs, notamment pour modifier les nuances ou les enrichir en reflets.

Les compositions de teinture contiennent également, dans leur forme de réalisation préférée, des agents tensioactifs anioniques, cationiques, non-ioniques, amphotères, bien connus de la technique, ou leurs mélanges, dans des proportions comprises entre 0,5 et 55 % en poids, et de préférence entre 2 et 50 % en poids par rapport au poids total de la composition.

Elles peuvent également contenir des solvants organiques. Parmi ceux-ci, on peut citer à titre d'exemple, les alcanols inférieurs en C₁-C₄, tels que l'éthanol et l'isopropanol, le glycérol, les glycols ou éthers de glycols comme le 2-butoxyéthanol, le propylèneglycol, le monoéthyléther et le monométhyléther du diéthylèneglycol, ainsi que les alcools aromatiques comme l'alcool benzylique ou le phénoxyéthanol, les produits analogues et leurs mélanges.

Ces solvants sont présents de préférence dans des proportions comprises entre 1 et 40 % en poids, et en particulier entre 5 et 30 % en poids par rapport au poids total de la composition.

On peut aussi ajouter des agents épaississants choisis par exemple parmi l'alginate de sodium, la gomme arabique, les polymères d'acide acrylique éventuellement réticulés, les dérivés de cellulose, les biopolysaccharides tels que la gomme de xanthane, ou des agents épaississants minéraux tels que la bentonite, présents de préférence dans des proportions comprises entre 0,1 et 5 % en poids, et en particulier entre 0,2 et 3 % en poids par rapport au poids total de la composition.

Des agents antioxydants peuvent également être introduits. Ils sont choisis en particulier parmi le sulfite de sodium, l'acide thioglycolique, l'acide thiolactique, le bisulfite de sodium, l'acide déhydroascorbique, l'hydroquinone, la 2-méthyl hydroquinone, la tertiobutyl hydroquinone et l'acide homogentisique et sont présents dans des proportions comprises entre 0,05 et 1,5 % en poids par rapport au poids total de la composition.

Les compositions de teinture peuvent également contenir d'autres adjuvants cosmétiquement acceptables, tels que par exemple des agents de pénétration, des agents séquestrants, des parfums, des tampons, des agents dispersants, des agents de traitement, des agents de conditionnement, des agents filmogènes, des agents conservateurs, des agents opacifiants.

La composition à appliquer sur les cheveux peut se présenter sous des formes diverses, telles que sous forme de liquide, de crème, de gel, ou sous toute autre forme appropriée pour réaliser une teinture des fibres kératiniques, et notamment des cheveux humains. Elle peut être conditionnée sous pression en flacon aérosol en présence d'un agent propulseur et former une mousse.

Des exemples concrets illustrant l'invention vont maintenant être donnés. On commencera par définir les tests utilisés pour évaluer les performances des teintures d'oxydation selon l'invention, de résistance à la transpiration, à la lumière et aux shampooings, aux intempéries ou à la permanente.

### Résistance à la transpiration :

On utilise une solution de sueur synthétique de composition suivante : 10 g de NaCl, 1 g d'hydrogénophosphate de potassium, 0,25 g d'histidine, de l'acide lactique jusqu'à pH = 3,2 et de l'eau distillée pour compléter à 100 g.

Dans un cristallisoir recouvert d'un verre de montre et renfermant cette solution de sueur, on immerge les mèches de cheveux teints qu'on laisse séjourner de 20 à 50 heures à 37° C. On rince ensuite les mèches et on les sèche.

### Résistance à la lumière (Xenotest) :

Les cheveux teints sont fixés sur un support (carton ou plastique). Ces supports sont disposés sur des porte-échantillons qui tournent autour d'une lampe Xénon pendant une durée variant de 20 à 80 heures sous un taux d'humidité variant de 25 à 75% HR (Humidité Relative) et à une température de 25° C.

### Résistance aux shampooings (machine Ahiba-Texomat) :

On place des mèches de cheveux teints dans un panier que l'on immerge dans une solution d'un shampooing standard. Le panier est soumis à un mouvement de va-et-vient vertical de fréquence variable ainsi qu'à un mouvement de rotation qui reproduisent l'action d'un frottement manuel, ce qui engendre la formation de mousse.

Après 3 minutes d'épreuve, on retire les mèches que l'on rince puis sèche. Les mèches teintes peuvent être soumises à plusieurs épreuves shampooings consécutives.

### Résistance aux intempéries (Epreuve combinée) :

Les mèches teintes sont exposées à la lumière forte ( Xenotest 40 h ), sous une humidité relative de 60 %, et simultanément, toutes les 12 heures, et pendant une durée de 20 minutes, elles sont soumises à une aspersion d'eau.

### Résistance à la permanente :

Les mèches teintes sont immergées dans une solution réductrice de permanente Dulcia Vital (L'Oréal), de force variant de 1 à 3, pendant une durée variant de 10 à 20 minutes; les mèches sont rincées; on les trempe ensuite dans une solution de fixateur (oxydant), pendant 5 minutes. Après rinçage à l'eau, lavage au shampooing standard et rinçage à l'eau, on les sèche.

### EXEMPLES 1 A 10 :

On a préparé 10 compositions de teinture, conformes à l'invention, suivantes :

| | |
|---|---|
| - Colorants (voir tableaux I et II) | x g |
| - Alcool oléique polyglycérolé à 2 moles de glycérol | 4,0 g |
| - Alcool oléique polyglycérolé à 4 moles de glycérol (78 % de MA) | 5,7 g MA |
| - Acide oléique | 3,0 g |
| - Amine oléique à 2 moles d'oxyde d'éthylène, vendue sous la dénomination Ethomeen 012 par la Société AKZO | 7,0 g |
| - Laurylaminosuccinamate de diéthylaminopropyle, sel de sodium à 55% de MA | 3,0 g MA |
| - Alcool oléique | 5,0 g |
| - Diéthanolamide d'acide oléique | 12,0 g |
| - Propylèneglycol | 3,5 g |
| - Alcool éthylique | 7,0 g |
| - Dipropylèneglycol | 0,5 g |
| - Monométhyléther de propylèneglycol | 9,0 g |
| - Métabisulfite de sodium en solution aqueuse à 35% de MA | 0,46 gMA |
| - Acétate d'ammonium | 0,8 g |
| - Antioxydant, séquestrant, | qs |
| - Parfum, conservateur, | qs |
| - Ammoniaque à 20% de NH₃ | 10,0 g |
| - Eau déminéralisée q.s.p. | 100 g |

Au moment de l'emploi, on a mélangé chacune de ces compositions poids pour poids avec de l'eau oxygénée titrant 20 volumes (6% en poids), de pH 3.

On a alors obtenu un mélange de pH 9,8

On a ensuite appliqué ce mélange sur des cheveux gris à 90% de blancs, naturels ou permanentés, pendant 30 minutes. Après rinçage, lavage au shampooing, rinçage et séchage, les cheveux sont ensuite teints dans les nuances allant du bleu au bleu-volet ou rouge-violet, chiffrées en valeurs MUNSELL (Norme ASTM D 1535-68) sur un colorimètre MINOLTA CM 2002, et dont les valeurs sont indiquées dans le tableau III donné ci-après.

Toutes ces nuances ont présenté de bonnes résistances, notamment au shampooing.

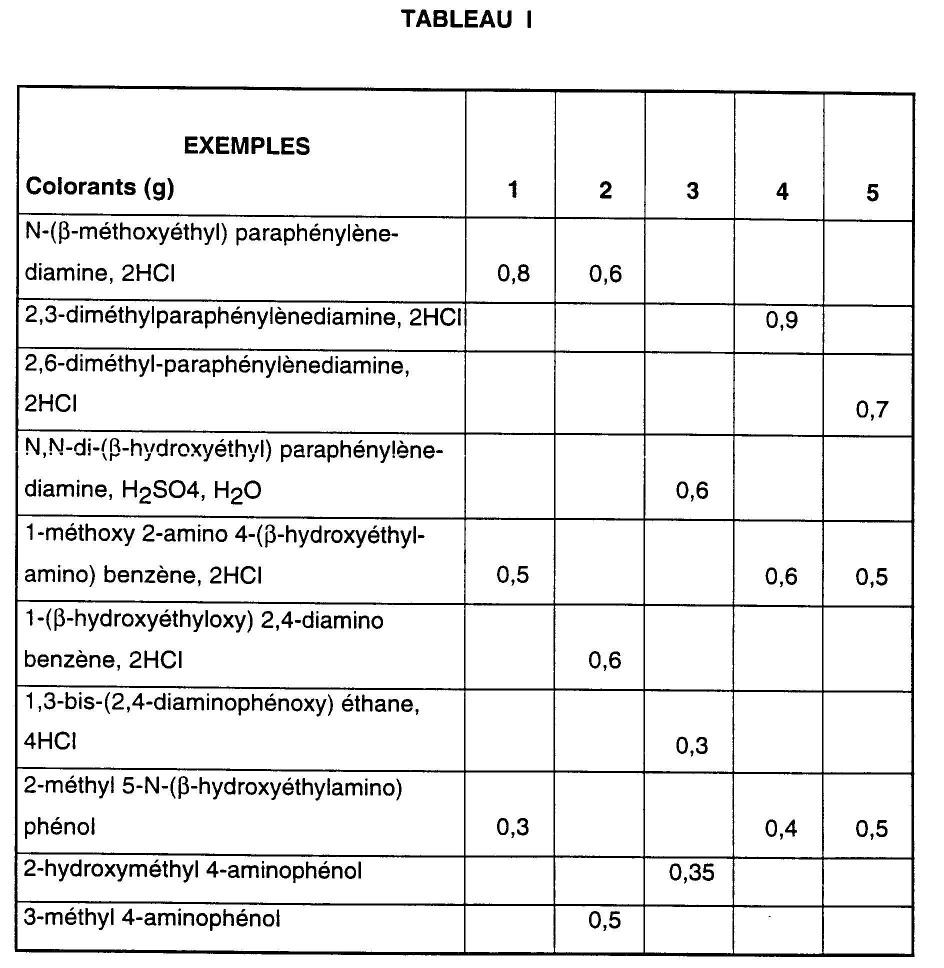

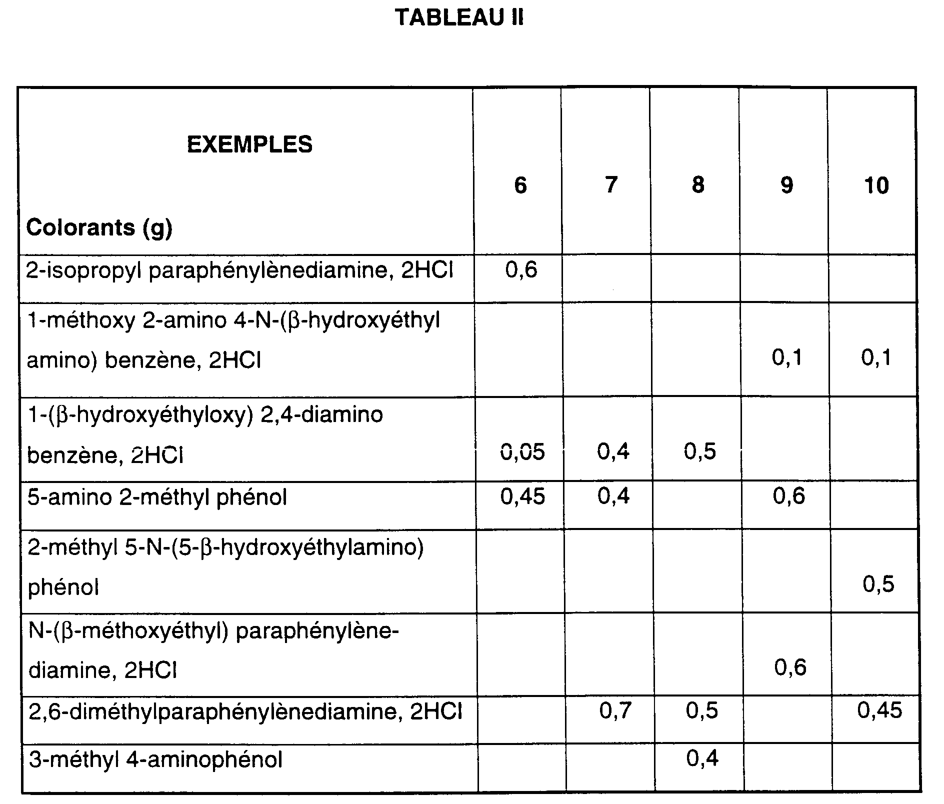

**TABLEAU III**

| **EXEMPLES** | **NUANCES MUNSELL MINOLTA CM 2002** | |
|---|---|---|
| | **cheveux naturels** | **cheveux permanentés** |
| 1 | 5,6 PB 2,6 / 2,4 | 7,2 PB 2,0 / 1,9 |
| 2 | 1,7 P 2,6 / 0,9 | 0,6 P 2,3 / 1,0 |
| 3 | 1,0 PB 3,3 / 1,0 | 4,6 PB 2,3 / 1,1 |
| 4 | 6,1 PB 2,8 / 1,5 | 7,0 PB 2,4 / 1,7 |
| 5 | 7,3 PB 2,4 / 1,8 | 8 PB 2,0 / 1,3 |
| 6 | 1,7 RP 3,2 / 1,6 | 9,7 P 2,4 / 1,8 |
| 7 | 8,0 PB 2,6 / 1,6 | 8,4 PB 2,0 / 1,3 |
| 8 | 2,4 P 2,6 / 1,2 | 2,1 P 2,0 / 1,0 |
| 9 | 2,3 P 2,8 / 1,9 | 9,9 PB 2,2 / 2,2 |
| 10 | 7,5 P 3,3 / 0,9 | 3,9 P 2,7 / 1,3 |

## Revendications

1. Composition de teinture d'oxydation pour fibres kératiniques, en particulier pour fibres kératiniques humaines telles que les cheveux, caractérisée par le fait qu'elle comprend, dans un milieu approprié pour la teinture :
(a) au moins un premier précurseur de colorant d'oxydation choisi parmi les paraphénylènediamines de formule (I) suivante : 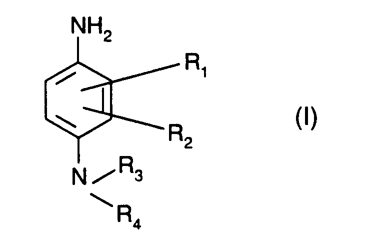 dans laquelle les radicaux R₁, R₂, R₃ et R₄ ont les significations soumises aux conditions suivantes :
- ou bien R₁ et R₂ représentent un atome d'hydrogène, lorsque R₃ et R₄, identiques, représentent un groupement -(CH₂)ₙ-OH, dans lequel n est égal à 2, 3 ou 4,
- ou bien R₁, R₂ et R₃ représentent un atome d'hydrogène, lorsque R₄ représente soit un groupement -(CH₂)ₙ-OR₅ dans lequel R₅ désigne un radical méthyle ou éthyle, avec n égal à 2 ou 3, soit un groupement mono-ou dihydroxypropyle,
- ou bien R₃ et R₄ représentent un atome d'hydrogène, lorsque R₁ et R₂, identiques, représentent un radical méthyle ou éthyle et sont situés en positions 2,3 ; 2,5 ou 2,6 sur le noyau benzénique,
- ou bien R₂, R₃ et R₄ représentent un atome d'hydrogène, lorsque R₁ représente un radical isopropyle,
et/ou au moins un des sels d'addition de ces composés de formule (I) avec un acide;
(b) au moins un premier coupleur de type métaphénylènediamine de formule (II) suivante : 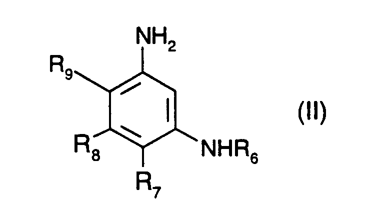 dans laquelle R₆ représente un atome d'hydrogène, un radical alkyle, mono-ou polyhydroxyalkyle, R₇ représente un atome d'hydrogène, un radical alkyle ou monohydroxyalcoxy, R₈ représente un atome d'hydrogène ou un radical alkyle, R₉ représente un radical alcoxy, aminoalcoxy, mono- ou poly-hydroxyalcoxy ou un radical 2,4-diaminophénoxyalcoxy, lesdits radicaux alkyle ou alcoxy ci-dessus contenant de 1 à 4 atomes de carbone, lesdits radicaux mono- ou polyhydroxyalkyle et mono- ou poly-hydroxyalcoxy ci-dessus désignant des radicaux alkyle ou alcoxy contenant de 2 à 3 atomes de carbone et comportant de 1 à 3 groupements hydroxyle, étant entendu qu'au moins un des radicaux R₇ ou R₈ désigne un atome d'hydrogène,
et/ou au moins l'un des sels d'addition de ces composés de formule (II) avec un acide;
(c)
- soit au moins un deuxième précurseur de colorant d'oxydation, de type paraaminophénol de formule (III) suivante : 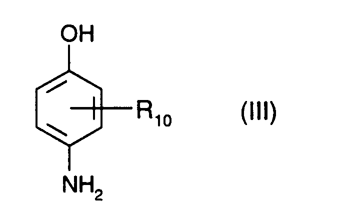 dans laquelle R₁₀ représente un radical alkyle, monohydroxyalkyle ou monoalcoxyalkyle, les radicaux alkyle et alcoxy ci-dessus contenant de 1 à 4 atomes de carbone,
et/ou au moins l'un des sels d'addition de ces composés de formule (III) avec un acide;
- soit au moins un deuxième coupleur, de type méta-aminophénol de formule (IV) suivante : 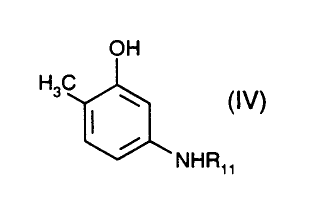 dans laquelle R₁₁ représente un atome d'hydrogène, un radical alkyle contenant 1 ou 2 atomes de carbone, un radical β-hydroxyalkyle contenant de 2 à 3 atomes de carbone,
et/ou au moins l'un des sels d'addition de ces composés de formule (IV) avec un acide ;
à l'exclusion :
(i) d'une composition renfermant simultanément des composés de formules (I), (II) et (IV) pour lesquelles R₁, R₂, désignent le radical méthyle en position 2 sur le noyau benzénique, R₃, R₄, R₆, R₇, R₈, désignent un atome d'hydrogène, R₉ désigne un radical aminoéthyloxy, et R₁₁, un groupement β-hydroxyéthyle,
et (ii) d'une composition renfermant simultanément des composés de formules (I), (II) et (IV) pour lesquelles R₁, R₂, R₃, R₆, R₇, R₈, R₁₁, désignent l'hydrogène, R₄ désigne un radical méthoxyéthyle, et R₉ désigne un groupement β-hydroxyéthyloxy.

2. Composition de teinture selon la revendication 1, caractérisée par le fait que la paraphénylènediamine de formule (I) est choisie parmi les composés suivants :
la 2,6-diméthylparaphénylènediamine ;
la 2,6-diéthylparaphénylènediamine ;
la 2,3-diméthylparaphénylènediamine ;
la 2,5-diméthylparaphénylènediamine ;
la 2-isopropylparaphénylènediamine ;
la N-(β-méthoxyéthyl) paraphénylènediamine ;
la N,N-di-(β-hydroxyéthyl) paraphénylènediamine
et leurs sels d'addition avec un acide.

3. Composition de teinture selon la revendication 1 ou 2, caractérisée par le fait que l'on utilise une métaphénylènediamine choisie parmi les composés de formule (II) dans laquelle R₆ désigne un atome d'hydrogène, un radical alkyle ou hydroxyalkyle, R₇ et R₈ désignent un atome d'hydrogène, R₉ désigne un radical alcoxy, aminoalcoxy, hydroxyalcoxy ou poly-hydroxyalcoxy, les radicaux alkyle et alcoxy de tous ces composés contenant de 1 à 4 atomes de carbone, à l'exception du groupement poly-hydroxyalcoxy pour lequel le radical alcoxy comporte de 2 à 4 atomes de carbone et de 1 à 3 radicaux hydroxyle.

4. Composition de teinture selon la revendication 1, 2 ou 3, caractérisée par le fait que la métaphénylènediamine de formule (II) est choisie parmi le 1-(β-hydroxyéthyloxy) 2,4-diamino benzène et le 1-méthoxy 2-amino 4-(β-hydroxyéthylamino) benzène et leurs sels d'addition avec un acide.

5. Composition de teinture selon l'une quelconque des revendications 1 à 4, caractérisée par le fait que l'on utilise un para-aminophénol de formule (III) dans laquelle le radical alkyle désigne méthyle, éthyle, propyle, isopropyle, butyle, secbutyle, tertiobutyle, et les radicaux monohydroxyalkyle et monoalcoxyalkyle désignent -CH₂OH ; -CH₂-CH₂OH ; -CH₂-CHOH-CH₂OH ; -CH₂-CHOH-CH₃ ou encore -CH₂ O CH₃.

6. Composition de teinture selon l'une quelconque des revendications 1 à 5, caractérisée par le fait que le para-aminophénol de formule (III) est choisi parmi le 3-méthyl 4-aminophénol, le 3-éthyl 4-aminophénol, le 3-hydroxyméthyl 4-aminophénol, le 2-méthyl 4-aminophénol, le 2-hydroxyméthyl 4-aminophénol, le 3-(méthoxyméthyl) 4-aminophénol, le 2-(méthoxyméthyl) 4-aminophénol et leurs sels d'addition avec un acide.

7. Composition de teinture selon l'une quelconque des revendications 1 à 6, caractérisée par le fait que l'on utilise un méta-aminophénol de formule (IV) dans laquelle le radical alkyle désigne méthyle ou éthyle, et le radical β-hydroxyalkyle désigne -CH₂-CH₂OH ou -CH₂-CH₂-CH₂OH.

8. Composition de teinture selon l'une quelconque des revendications 1 à 7, caractérisée par le fait que le méta-aminophénol de formule (IV) est choisi dans le groupe constitué par le 2-méthyl 5-aminophénol, le 2-méthyl 5-N-méthylaminophénol, le 2-méthyl 5-N-éthylaminophénol, le 2-méthyl 5-N-(β-hydroxyéthylamino) phénol, le 2-méthyl 5-N-(β-hydroxypropylamino) phénol et leurs sels d'addition avec un acide.

9. Composition de teinture selon l'une quelconque des revendications 1 à 8, caractérisée par le fait que les sels d'addition avec un acide sont choisis parmi les chlorhydrates, les sulfates, les bromhydrates et les tartrates.

10. Composition de teinture selon l'une quelconque des revendications 1 à 9, caractérisée par le fait que le premier précurseur de colorant d'oxydation de formule (I) est présent dans une proportion pondérale comprise entre 0,01 et 10% environ par rapport au poids total de la composition, le premier coupleur de formule (II) y est présent dans une proportion comprise entre 0,001 et 3% environ, le second précurseur de colorant d'oxydation de formule (III) y est présent entre 0,01 et 5% environ ou le second coupleur de formule (V), entre 0,005 et 5% environ.

11. Composition de teinture selon l'une quelconque des revendications 1 à 10, prête à l'emploi, caractérisée par le fait qu'elle contient en outre un agent oxydant et qu'elle possède un pH compris entre 3 et 11.

12. Procédé de teinture des fibres kératiniques et en particulier des fibres kératiniques humaines telles que les cheveux, caractérisé par le fait qu'il consiste à appliquer sur les fibres une composition de teinture (A) selon l'une quelconque des revendications 1 à 11, et à révéler la couleur en milieu alcalin neutre ou acide à l'aide d'un agent oxydant qui est ajouté juste au moment de l'emploi à cette composition (A) ou qui est présent dans une composition (B) appliquée simultanément ou séquentiellement de façon séparée.

13. Dispositif à plusieurs compartiments ou "kit" pour la teinture des fibres kératiniques et en particulier des fibres kératiniques humaines telles que les cheveux, caractérisé par le fait qu'il comporte au moins deux compartiments dont l'un d'entre eux renferme une composition (A) telle que définie dans l'une quelconque des revendications 1 à 11 et un autre une composition (B) comprenant un agent oxydant dans un milieu approprié pour la teinture.

14. Utilisation d'une composition de teinture selon l'une quelconque des revendications 1 à 11 ou d'un dispositif de teinture ou "kit" à plusieurs compartiments selon la revendication 13 pour la teinture des fibres kératiniques, en particulier des fibres kératiniques humaines telles que les cheveux.

## Claims

1. Oxidation dye composition for keratinous fibres, in particular for human keratinous fibres such as hair, characterized in that it comprises, in a medium which is suitable for dyeing:
(a) at least a first oxidation dye precursor chosen from the para-phenylenediamines of the following formula (I) 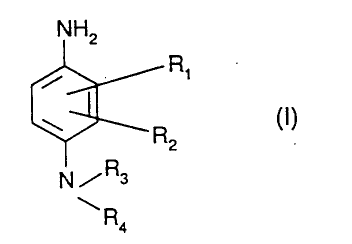 in which the radicals R₁, R₂, R₃ and R₄ have the meanings with the following provisos:
- either R₁ and R₂ represent a hydrogen atom when R₃ and R₄, which are identical, represent a group -(CH₂)ₙ-OH in which n is equal to 2, 3 or 4,
- or R₁, R₂ and R₃ represent a hydrogen atom when R₄ either represents a group -(CH₂)ₙ-OR₅, in which R₅ denotes a methyl or ethyl radical, with n equal to 2 or 3, or represents a mono- or dihydroxypropyl group,
- or R₃ and R₄ represent a hydrogen atom when R₁ and R₂, which are identical, represent a methyl or ethyl radical and are situated in positions 2,3; 2,5 or 2,6 on the benzene ring,
- or R₂, R₃ and R₄ represent a hydrogen atom when R₁ represents an isopropyl radical,
and/or at least one of the addition salts of these compounds of formula (I) with an acid;
(b) at least a first coupling agent of metaphenylenediamine type, of the following formula (II): 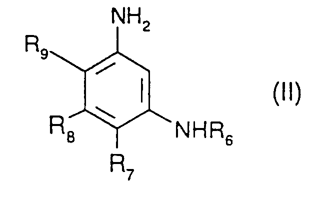 in which R₆ represents a hydrogen atom, an alkyl radical or a mono- or polyhydroxyalkyl radical, R₇ represents a hydrogen atom, an alkyl radical or a monohydroxyalkoxy radical, R₈ represents a hydrogen atom or an alkyl radical, R₉ represents an alkoxy radical, an aminoalkoxy radical, a mono- or polyhydroxyalkoxy radical or a 2,4-diaminophenoxyalkoxy radical, the abovesaid alkyl or alkoxy radicals containing from 1 to 4 carbon atoms, the abovesaid mono- or polyhydroxyalkyl radicals and mono- or polyhydroxyalkoxy radicals denoting alkyl or alkoxy radicals containing from 2 to 3 carbon atoms and including from 1 to 3 hydroxyl groups, it being understood that at least one of the radicals R₇ or R₈ denotes a hydrogen atom,
and/or at least one of the addition salts of these compounds of formula (II) with an acid;
(c)
- either at least a second oxidation dye precursor, of para-aminophenol type, of following formula (III): 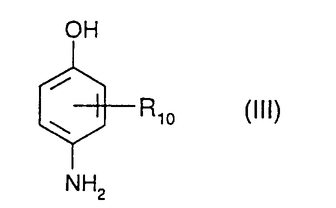 in which R₁₀ represents an alkyl, monohydroxyalkyl or monoalkoxyalkyl radical, the alkyl and alkoxy radicals above containing from 1 to 4 carbon atoms,
and/or at least one of the addition salts of these compounds of formula (III) with an acid;
- or at least a second coupling agent, of metaaminophenol type, of following formula (IV): 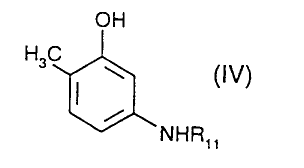 in which R₁₁ represents a hydrogen atom, an alkyl radical containing 1 or 2 carbon atoms or a β-hydroxyalkyl radical containing from 2 to 3 carbon atoms,
and/or at least one of the addition salts of these compounds of formula (IV) with an acid;
with the exclusion of
(i) a composition which simultaneously contains compounds of formulae (I), (II) and (IV) for which R₁ and R₂ denote a methyl radical in position 2 on the benzene ring, R₃, R₄, R₆, R₇ and R₈ denote a hydrogen atom, R₉ denotes an aminoethyloxy radical, and R₁₁ a β-hydroxyethyl group,
and (ii) a composition which simultaneously contains compounds of formulae (I), (II) and (IV) for which R₁, R₂, R₃, R₆, R₇, R₈ and R₁₁ denote hydrogen, R₄ denotes a methoxyethyl radical, and R₉ denotes a β-hydroxyethyloxy group.

2. Dye composition according to Claim 1, characterized in that the para-phenylenediamine of formula (I) is chosen from the following compounds:
2,6-dimethyl-para-phenylenediamine;
2,6-diethyl-para-phenylenediamine;
2,3-dimethyl-para-phenylenediamine;
2,5-dimethyl-para-phenylenediamine;
2-isopropyl-para-phenylenediamine;
N-(β-methoxyethyl)-para-phenylenediamine;
N,N-di(β-hydroxyethyl)-para-phenylenediamine, and the addition salts thereof with an acid.

3. Dye composition according to Claim 1 or 2, characterized in that a meta-phenylenediamine is used which is chosen from the compounds of formula (II) in which R₆ denotes a hydrogen atom, an alkyl radical or a hydroxyalkyl radical, R₇ and R₈ denote a hydrogen atom, R₉ denotes an alkoxy radical, an aminoalkoxy radical, a hydroxyalkoxy radical or a polyhydroxyalkoxy radical, the alkyl and alkoxy radicals of all these compounds containing from 1 to 4 carbon atoms, with the exception of the polyhydroxyalkoxy group for which the alkoxy radical is composed of from 2 to 4 carbon atoms and of from 1 to 3 hydroxyl radicals.

4. Dye composition according to Claim 1, 2 or 3, characterized in that the meta-phenylenediamine of formula (II) is chosen from 1-(β-hydroxyethyloxy)-2,4-diaminobenzene and 1-methoxy-2-amino-4-(β-hydroxy-ethylamino)benzene and the addition salts thereof with an acid.

5. Dye composition according to any one of Claims 1 to 4, characterized in that a para-aminophenol of formula (III) is used in which the alkyl radical denotes methyl, ethyl, propyl, isopropyl, butyl, sec-butyl or tert-butyl and the monohydroxyalkyl and monoalkoxyalkyl radicals denote -CH₂OH; -CH₂-CH₂OH; -CH₂-CHOH-CH₂OH; -CH₂-CHOH-CH₃ or alternatively -CH₂OCH₃.

6. Dye composition according to any one of Claims 1 to 5, characterized in that the para-aminophenol of formula (III) is chosen from 3-methyl-4-aminophenol, 3-ethyl-4-aminophenol, 3-hydroxymethyl-4-aminophenol, 2-methyl-4-aminophenol, 2-hydroxymethyl-4-aminophenol, 3-methoxymethyl-4-aminophenol, 2-methoxymethyl-4-aminophenol, and the addition salts thereof with an acid.

7. Dye composition according to any one of Claims 1 to 6, characterized in that a meta-aminophenol of formula (IV) is used in which the alkyl radical denotes methyl or ethyl, and the β-hydroxyalkyl radical denotes -CH₂-CH₂OH or -CH₂-CH₂-CH₂OH.

8. Dye composition according to any one of Claims 1 to 7, characterized in that the meta-aminophenol of formula (IV) is chosen from the group consisting of 2-methyl-5-aminophenol, 2-methyl-5-N-methylaminophenol, 2-methyl-5-N-ethylaminophenol, 2-methyl-5-N-(βhydroxyethylamino)phenol, 2-methyl-5-N-(β-hydroxypropylamino)phenol, and the addition salts thereof with an acid.

9. Dye composition according to any one of Claims 1 to 8, characterized in that the addition salts with an acid are chosen from hydrochlorides, sulphates, hydrobromides and tartrates.

10. Dye composition according to any one of Claims 1 to 9, characterized in that the first oxidization dye precursor of formula (I) is present in a weight proportion of between 0.01 and 10% approximately relative to the total weight of the composition, the first coupling agent of formula (II) is present therein in a proportion of between 0.001 and 3% approximately, the second oxidation dye precursor of formula (III) is present therein at between 0.01 and 5% approximately or the second coupling agent of formula (V) is present therein at between 0.005 and 5% approximately.

11. Dye composition according to any one of Claims 1 to 10, which is in a ready-to-use form, characterized in that it additionally contains an oxidizing agent and in that it has a pH between 3 and 11.

12. Process for dyeing keratinous fibres and in particular human keratinous fibres such as hair, characterized in that it consists in applying to the fibres a dye composition (A) according to any one of Claims 1 to 11, and in developing the colour in an alkaline, neutral or acidic medium using an oxidizing agent which is added to this composition (A) only at the time of use or which is present in a composition (B) that is applied simultaneously or sequentially in a separate manner.

13. Device containing several compartments, or "kit", for dyeing keratinous fibres and in particular human keratinous fibres such as hair, characterized in that it is composed of at least two compartments, one of which contains a composition (A) as defined in any one of Claims 1 to 11, and another of which contains a composition (B) comprising an oxidizing agent in a medium which is suitable for dyeing.

14. Use of a dye composition according to any one of Claims 1 to 11 or of a dyeing device or "kit" containing several compartments according to Claim 13, for dyeing keratinous fibres, in particular human keratinous fibres such as hair.

## Patentansprüche

1. Zusammensetzung zum oxidativen Färben von Keratinfasern und insbesondere von menschlichen Keratinfasern, wie dem Haar,
dadurch gekennzeichnet, daß
sie in einem zum Färben geeigneten Medium enthält:
(a) mindestens ein erstes Farbstoffvorprodukt eines Oxidationsfarbstoffes, das unter p-Phenylendiaminen der folgenden Formel (I) ausgewählt ist: 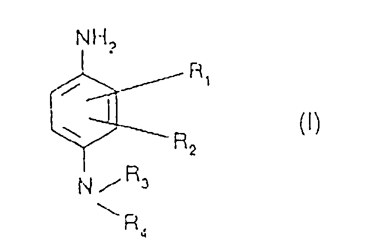 worin die Gruppen R₁, R₂, R₃ und R₄ mit folgenden Maßgaben bedeuten:
- entweder bedeuten R₁ und R₂ ein Wasserstoffatom, wenn R₃ und R₄ gleichzeitig eine Gruppe -(CH₂)ₙ-OH bedeuten, wobei n gleich 2, 3 oder 4 ist,
- oder R₁, R₂ und R₃ bedeuten ein Wasserstoffatom, wenn R₄ entweder eine Gruppe -(CH₂)ₙ-OR₅ (worin R₅ eine Methyl- oder Ethylgruppe bedeutet und n gleich 2 oder 3 ist) oder eine Mono- oder Dihydroxypropylgruppe bedeutet,
- oder R₃ und R₄ bedeuten ein Wasserstoffatom, wenn R₁ und R₂ beide eine Methyl- oder Ethylgruppe bedeuten und am Benzolring in 2,3-, 2,5- oder 2,6-Position substituiert sind,
- oder R₂, R₃ und R₄ bedeuten ein Wasserstoffatom, wenn R₁ eine Isopropylgruppe ist,
und/oder mindestens ein Additionssalz dieser Verbindungen der Formel (I) mit einer Säure;
(b) mindestens einen ersten Kuppler vom Typ m-Phenylendiamin der folgenden Formel (II): 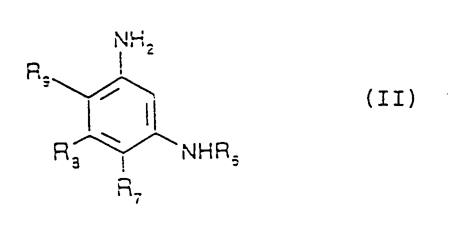 worin bedeuten:
R₆: Wasserstoff, Alkyl, Mono- oder Polyhydroxyalkyl,
R₇: Wasserstoff, Alkyl oder Monohydroxyalkoxy,
R₈: Wasserstoff oder Alkyl
R₉: Alkoxy, Aminoalkoxy, Mono- oder Polyhydroxyalkoxy oder 2,4-Diaminophenoxyalkoxy,
wobei die obengenannten Alkyl- oder Alkoxygruppen 1 bis 4 Kohlenstoffatome enthalten, und die obengenannten Mono- oder Polyhydroxyalkylgruppen und Mono- oder Polyhydroxyalkoxygruppen Alkyl- oder Alkoxygruppen mit 2 bis 3 Kohlenstoffatomen enthalten und 1 bis 3 Hydroxygruppen aufweisen, mit der Maßgbe, daß mindestens eine der Gruppen R₇ oder R₈ ein Wasserstoffatom bedeutet,
und/oder mindestens ein Additionssalz dieser Verbindungen der Formel (II) mit einer Säure;
(c)
- entweder mindestens ein zweites Farbstoffvorprodukt einer Oxidationsfarbe vom Typ p-Aminophenol der nachfolgenden Formel (III): 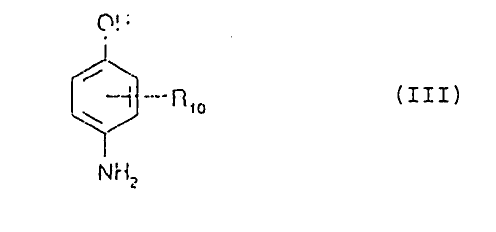 worin R₁₀ bedeutet:
Alkyl, Monohydroxyalkyl oder Monoalkoxyalkyl, wobei die vorgenannten Alkyl- und Alkoxygruppen 1 bis 4 Kohlenstoffatome enthalten,
und/oder mindestens ein Additionssalz dieser Verbindungen der Formel (III) mit einer Säure
- oder mindestens einen zweiten Kuppler vom Typ m-Aminophenol der folgenden Formel (IV): 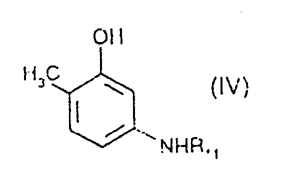 worin R₁₁ bedeutet:
Wasserstoff, Alkyl mit 1 bis 2 Kohlenstoffatomen, β-Hydroxyalkyl mit 2 bis 3 Kohlenstoffatomen,
und/oder mindestens ein Additionssalz dieser Verbindungen der Formel IV mit einer Säure,
wobei ausgeschlossen sind:
(i) eine Zusammensetzung, die gleichzeitig die Verbindungen der Formeln (I), (II) und (IV) enthält, worin R₁ und R₂ eine Methylgruppe in 2-Position am Benzolring, R₃, R₄, R₆, R₇ und R₈ ein Wasserstoffatom, R₉ eine Aminoethyloxygruppe und R₁₁ eine β-Hydroxyethylgruppe bedeuten,
und
(ii) eine Zusammensetzung, die gleichzeitig die Verbindungen der Formeln (I), (II) und (IV) enthält, worin R₁, R₂, R₃, R₆, R₇, R₈ und R₁₁ Wasserstoff, R₄ eine Methoxyethylgruppe und R₉ ein β-Hydroxyethyloxygruppe bedeuten.

2. Zusammensetzung zum Färben nach Anspruch 1, dadurch gekennzeichnet, daß das p-Phenylendiamin der Formel (I) unter folgenden Verbindungen ausgewählt ist:
2,6-Dimethyl-p-phenylendiamin,
2,6-Diethyl-p-phenylendiamin,
2,3-Dimethyl-p-phenylendiamin,
2,5-Dimethyl-p-phenylendiamin,
2-Isopropyl-p-phenylendiamin,
N-(β-methoxyethyl)-p-phenylendiamin,
N,N-di-(β-hydroxyethyl)-p-phenylendiamin und
den Additionssalzen dieser Verbindungen mit einer Säure.

3. Zusammensetzung zum Färben nach Anspruch 1 oder 2, dadurch gekennzeichnet, daß ein m-Phenylendiamin verwendet wird, das unter Verbindungen der Formel (II) ausgewählt ist, worin R₆ Wasserstoff, Alkyl oder Hydroxyalkyl, R₇ und R₈ Wasserstoff, und R₉ Alkoxy, Aminoalkoxy, Hydroxyalkoxy oder Polyhydroxyalkoxy bedeuten, wobei alle Alkyl- und Alkoxygruppen dieser Verbindungen 1 bis 4 Kohlenstoffatome enthalten, mit Ausnahme der Polyhydroxyalkoxygruppe, deren Alkoxygruppe 2 bis 4 Kohlenstoffatome und 1 bis 3 Hydroxygruppen enthält.

4. Zusammensetzung zum Färben nach Anspruch 1, 2 oder 3, dadurch gekennzeichnet, daß das m-Phenylendiamin der Formel (II) unter 1-(β-Hydroxyethyloxy)-2,4-diaminobenzol, 1-Methoxy-2-amino-4-(β-hydroxyethylamino)-benzol und den Additionssalzen dieser Verbindungen mit einer Säure ausgewählt ist.

5. Zusammensetzung zum Färben nach einem der Ansprüche 1 bis 4, dadurch gekennzeichnet, daß ein p-Aminophenol der Formel (III) verwendet wird, worin die Alkylgruppe Methyl, Ethyl, Propyl, Isopropyl, Butyl, sek. -Butyl, tert.-Butyl und die Monohydroxyalkyl- und Monoalkoxyalkylgruppen -CH₂OH, -CH₂₋CH₂OH, -CH₂-CHOH-CH₂OH, - CH₂-CHOH-CH₃ oder auch -CH₂OCH₃ bedeuten.

6. Zusammensetzung zum Färben nach einem der Ansprüche 1 bis 5, dadurch gekennzeichnet, daß daß p-Aminophenol der Formel (III) unter 3-Methyl-4-aminophenol, 3-Ethyl-4-aminophenol, 3-Hydroxymethyl-4-aminophenol, 2-Methyl-4-aminophenol, 2-Hydroxyethyl-4-aminophenol, 3-(Methoxymethyl)-4-aminophenol, 2-(Methoxymethyl)-4-aminophenol und den Additionssalzen dieser Verbindungen mit einer Säure ausgewählt ist.

7. Zusammensetzung zum Färben nach einem der Ansprüche 1 bis 6, dadurch gekennzeichnet, daß ein m-Aminophenol der Formel (IV) verwendet wird, worin die Alkylgruppe Methyl oder Ethyl und die β-Hydroxyalkylgruppe -CH₂-CH₂OH oder -CH₂-CH₂-CH₂OH bedeuten.

8. Zusammensetzung zum Färben nach einem der Ansprüche 1 bis 7, dadurch gekennzeichnet, daß das m-Aminophenol der Formel (IV) unter 2-Methyl-5-aminophenol, 2-Methyl-5-N-methylaminophenol, 2-Methyl-5-N-ethylaminophenol, 2-Methyl-5-N-(β-Hydroxyethylamino)-phenol, 2-Methyl-5-N-(β-hydroxypropylamino)-phenol und den Additionssalzen dieser Verbindungen mit einer Säure ausgewählt ist.

9. Zusammensetzung zum Färben nach einem der Ansprüche 1 bis 8, dadurch gekennzeichnet, daß die Additionssalze mit einer Säure unter Hydrochloriden, Sulfaten, Hydrobromiden und Tartraten ausgewählt sind.

10. Zusammensetzung zum Färben nach einem der Ansprüche 1 bis 9, dadurch gekennzeichnet, daß das erste Farbstoffvorprodukt einer Oxidationsfarbe der Formel (I) in einem Anteil von etwa 0,01 bis 10 Gew.-%, bezogen auf das Gesamtgewicht der Zusammensetzung, der erste Kuppler der Formel (II) in einem Anteil von etwa 0,001 bis 3 Gew.-%, das zweite Farbstoffvorprodukt einer Oxidationsfarbe der Formel (II) in einem Anteil von etwa 0,01 bis 5 Gew.-% und der zweite Kuppler der Formel (IV) in einem Anteil von etwa 0,005 bis 5 Gew.-% vorliegen.

11. Zusammensetzung zum Färben nach einem der Ansprüche 1 bis 10, die anwendungsfertig ist, und dadurch gekennzeichnet ist, daß sie ferner ein Oxidationsmittel enthält und einem pH-wert im Bereich von 3 bis 11 aufweist.

12. Verfahren zum Färben von Keratinfasern und insbesondere von menschlichen Keratinfasern, wie dem Haar, dadurch gekennzeichnet, daß es darin besteht, auf die Fasern eine Zusammensetzung zum Färben (A) nach einem der Ansprüche 1 bis 11 aufzutragen, die Farbe in einem alkalischen, neutralen oder sauren Medium mit einem Oxidationsmittel zu entwickeln, das der Zusammensetzung (A) unmittelbar bei der Anwendung zugesetzt wird oder in eine Zusammensetzung (B) vorliegt, die gleichzeitig oder getrennt davon anschließend aufgetragen wird.

13. Vorrichtung mit mehreren Anteilungen oder oder "Kit" zum Färben von Keratinfasern und insbesondere von menschlichen Keratinfasern, wie dem Haar, dadurch gekennzeichnet, daß sie mindestens zwei Abteilungen enthält, wobei eine der Abteilungen eine Zusammensetzung (A) nach einem der Ansprüche 1 bis 11, und eine weitere Abteilung eine Zusammensetzung (B) enthalten, die in einem zum Färben geeigneten Medium ein Oxidationsmittel enthält.

14. Verwendung einer Zusammensetzung zum Färben nach einem der Ansprüche 1 bis 11 oder einer Vorrichtung zum Färben oder eines "Kits" mit mehreren Abteilungen nach Anspruch 13 zum Färben von Keratinfasern und insbesondere von menschlichen Keratinfasern, wie dem Haar.
